# EUROPEAN PATENT APPLICATION

(11) **EP 3 072 498 A1**
(43) Date of publication of application: **28.09.2016**
(21) Application number: 15160912.0
(22) Date of filing: 25.03.2015
(51) Int. Cl.: A61K 6/00, A61K 6/083, C08L 33/14

(54) **Dental composites comprising porous fillers**

(71) Applicant: Septodont ou Septodont SAS ou Specialites Septodont, 94100 Saint Maur des Fossés (FR)
(72) Inventor: Hallay, Franck, 69300 Caluire et Cuire (FR); Colon, Pierre, 94100 Saint Maur des Fossés (FR); Richard, Gilles, 91560 Crosnes (FR); Bois, Laurence, 69004 Lyon (FR); Brioude, Arnaud, 69100 Villeurbanne (FR); Grosgogeat, Brigitte, 69500 Bron (FR)
(74) Representative: Icosa

(57) **Abstract**

The invention relates to a composition in the dental field. Especially, the invention relates to a composition resulting from the mixture of:
- a resin matrix; and
- a filler blend comprising at least one primary filler and at least one porous filler.

The invention also relates to a method for preparing said dental composition and its uses in the dental field. The invention also relates to a process for manufacturing a composite comprising the preparation of the composition of the invention. The present invention also relates to a kit of parts for the preparation of a composition suitable in dentistry comprising in a first container a resin matrix according to the invention and in a second container a filler blend comprising at least one primary filler and at least one porous filler.

## Description

### FIELD OF INVENTION

The invention relates to a composition in the dental field. More precisely, the invention relates to a composition having a high filler content while keeping good handling properties. The present invention also relates to a method for preparing said composition. The present invention also concerns uses of said composition in the dental field; especially for providing dental composites with improved physical properties *(i.e.* polishability and mechanical properties) with good handling properties *(i.e.* stickiness, viscosity and thixotropy).

### BACKGROUND OF INVENTION

Composites are heterogeneous materials resulting from an intimate blend between at least two immiscible components: the reinforcement, ensuring the mechanical strength of the material, and the matrix (usually a polymer matrix), ensuring the cohesion of the structure and the transmission of efforts towards reinforcement.

This kind of materials has a particular structural organization giving to the composite improved properties with regard to those of individual components. The ability to obtain materials featuring high levels of performance has led many manufacturers to focus on composites.

In the dental field, composites are mainly used in order to replace amalgam; especially, for sealing or restoring teeth.

Compared with amalgam, the main asset of composite is an aesthetic advantage as it allows imitating the white color of the teeth. In addition, it is a more conservative treatment than amalgam.

Basically, a dental composite is a mixture of silicate glass particles within an acrylic monomer that is polymerized during or just before use. In particular, dental composites are made of four major components which are: resin matrix (or polymer matrix), fillers, a coupling agent and an initiator-accelerator system to cure the composition.

The mechanical properties of composites (hardness, durability ...) depend on the nature and the content of fillers in the material.

For improving mechanical properties of dental composites, there is a need to reach highest level of fillers content. However, the enhancement of fillers content leads to an increase of the viscosity and the consistency of final composite such as it is difficult for the practitioner to easily handle the dental composition before polymerization.

US 8,513,326 relates to dental compositions with improved mechanical properties. Especially, in US 8,513,326, the aim is to provide dental resins while avoiding issues linked to polymerization of conventional monomers used in dentistry (low conversion, excessive volumetric shrinkage, poor toughness and excessive water uptake). In US 8,513,326, it is mentioned that the amount of shrinkage can be reduced in some extent by increasing filler content. However, it is also mentioned that a high amount of fillers makes it difficult to have them mixed with the organic resin. Thus, to overcome these issues, Trujillo-Lemon et al. has developed carbamate-methacrylate monomers which allows 1) achieving higher conversion of monomers and 2) reducing volumetric shrinkage during the polymerization reaction. However, no dental composite was manufactured with these carbamate-methacrylate monomers; especially, the mixture with a filler blend comprising porous fillers was not provided. Furthermore, US 8,513,326 does not focus on the enhancement of the handling properties.

Thus, there is a need for providing composites having higher fillers content compared to classical composites, without compromising the handling properties; especially, allowing avoiding to get a too viscous and too sticky composite.

Surprisingly, the Applicant has shown that introducing a low content of porous fillers in a composition comprising carbamate-alkylacrylate-based resin matrix, results in achieving a high total filler content without increasing the viscosity and/or the consistency of the formulation before polymerization, and in a good handling by reduced stickiness, enhanced durability and glossy aspect of the final material.

### SUMMARY

This invention thus relates to a composition resulting from the mixture of:
- a resin matrix comprising:
   ▪ from 57% to 99%w/w of a mixture of photopolymerizable monomers comprising at least one carbamate-alkylacrylate monomer;
   ▪ from more than 0% to 42%w/w of at least one plasticizer;
   ▪ from more than 0% to 2%w/w of at least one polymerization inhibitor ;
   ▪ from more than 0% to 3% w/w of at least one photoiniator;
      in weight of the total weight of the resin matrix;
- and a filler blend comprising:
   ▪ at least one primary filler selected from mineral compounds; and
   ▪ from more than 0 to 30%w/w at least one porous filler, preferably selected from porous silica particles, more preferably selected from SBA-15, MCM-48, MSU-H and MCM-41;
      in weight of the total weight of the filler blend;
   wherein
   the ratio between the resin matrix and the filler blend ranges from about 0.25 to 2.5, preferably is 0.33;
   the porous filler has a size ranging from 20nm to 20µm and a pore size ranging from 2nm to 30nm.

According to one embodiment, the composition does not comprise any solvent.

According to one embodiment, the resin matrix further comprises at least one monomer selected from the group comprising at least one acrylate, alkylacrylate, methacrylate, styrenic, vinylic, allylic, oxetan, alkyloxetan, cyclic ether, acrylamide, methacrylamide and derivatives thereof.

According to one embodiment, the plasticizer is selected from compounds having at least two methacrylate functions and no carbamate function; preferably the plasticizer is 1,6 hexanediol dimethacrylate (HDDMA) or trimethylpropyltrimethacrylate (TMPTMA).

According to one embodiment, the polymerization inhibitor is butylhydroxytoluene.

According to one embodiment, the photoinitiator is a mixture of 2,4-dihydroxybenzophenone, ethyl 4-dimethylaminobenzoate and camphorquinone.

According to one embodiment, the primary filler is a Schott Glass^{®}; preferably a barium glass, an ytterbium glass or a mixture thereof.

According to one embodiment, the porous filler is functionalized by at least one silane bearing one or more functional or hydrophobic groups; preferably said groups are selected from methacryloyl, methacryloylalkyl, aminoalkyl; alkoxy, vinyl, allyl, aryl or alkyl.

The present invention also relates to a method of preparing the composition of the invention, comprising a step of mixing of a resin matrix and a filler blend, wherein the resin matrix comprises:
- from more than 0% to 2% w/w of at least one polymerization inhibitor ;
- from more than 0% to 3% w/w of photoinitiator in weight of the total resin matrix;
- from more than 0% to 42% w/w of at least one plasticizer, and
- from 57% to 99% w/w of a mixture of photopolymerizable monomers comprising carbamate-alkylacrylate monomers in weight of the total resin matrix;
and the filler blend comprises at least one primary filler and at least one porous filler;
wherein
said porous filler is preferably selected from porous silica particles, more preferably selected from SBA, MCM-48, MSU-H and MCM-41;
said porous filler has a size ranging from 20nm to 20µm and a pore size ranging from 2nm to 30nm;
the content of porous filler is ranging from more than 0% to 30%, preferably from 1 to 3% in weight of the total weight of the filler blend in the composition.

The invention also relates to a process for manufacturing a composite comprising:
(a) preparing the composition of the invention;
(b) molding the composition resulting from the step (a);
(c) implementing photopolymerization.

According to one embodiment, the photopolymerization is carried out during a time ranging from 5 to 60s, preferably ranging from 10s to 40s; more preferably during 20s.

The present invention also relates to a kit of parts for the preparation of a composition suitable for use in dentistry, comprising in a first container a resin matrix and a second container a filler blend comprising at least one primary filler and at least one porous filler.

The present invention also relates to a composition for use in dental sealing and/or restoration. In one embodiment, the composition is for use in the manufacture of a dental composite.

### DEFINITIONS

In the present invention, the following terms have the following meanings:
- **"About"** preceding a figure means plus or less 10% of the value of said figure;
- **"Acrylate":** refers to compound comprising a CH₂=CH(COO-) group;
- **"Alkoxy"** refers any group -O-alkyl, wherein alkyl is as defined above. Suitable alkoxy groups include for example methoxy, ethoxy, n-propoxy, isopropoxy, n-butoxy, f-butoxy, sec-butoxy, and n-pentoxy;
- **"Allyl"** or **"Allylic"** refers to the group CH₂=CH-CH₂-;
- **"Alkylacrylate":** refers to compound comprising a group CH₂=CR(COO-) wherein R is a C1-C10 alkyl group; in the present invention, R is preferably a methyl group (methacrylate compound) or an ethyl group (ethacrylate compound);
- **"Alkyl":** refers to any saturated linear or branched hydrocarbon chain, with 1 to 30 carbon atoms, preferably 1 to 20 carbon atoms, and more preferably methyl, ethyl, propyl, isopropyl, n-butyl, sec-butyl, isobutyl, tert-butyl, pentyl, hexyl, heptyl, octyl, nonyl, decyl, undecyl, dodecyl, tridecyl, tetradecyl, pentadecyl, cetyl, heptadecyl, octadecyl, nonadecyl and eicosyl;
- **"Aryl"** refers to a polyunsaturated, aromatic hydrocarbyl group having a single ring or multiple aromatic rings fused together (such as naphthyl) or linked covalently, typically containing 5 to 20, and preferably 6 to 12, carbon atoms having one or more aromatic rings among which it is possible to cite the phenyl group, the biphenyl group, the 1-naphthyl group, the 2-naphthyl group, the tetrahydronaphthyl group, the indanyl group and the binaphthyl group;
- **"Carbamate":** refers to compound comprising a group -O-CO-NH-;
- **"Composite":** refers to an assembly of at least one two non-miscible components having complementary properties. In the present invention, "composite" refers to the assembly between a polymerized resin matrix and a filler blend;
- **"Monomer":** refers to a molecule that forms the basic repeating unit for polymer;
- **"Oxetan":** refers to a cyclic ether comprising 3 carbon atoms and one oxygen atom;
- **"Photoinitiator"** refers to a light-sensitive compound used to initiate a radical polymerization reaction by irradiation;
- **"Photopolymerizable"** refers to a compound comprising at least one polymerizable function and wherein the polymerization is initiated by irradiation of said polymerizable function;
- **"Plasticizer":** refers to a compound or an additive that increases the plasticity or the fluidity of a composition or a material;
- **"Polymerization inhibitor":** refers to a chemical compound able to stop or to delay the polymerization reaction; in the present invention, the polymerization inhibitor is used in order to delay the photopolymerization when the sample is exposed at light or at a specific wavelength at the beginning of the polymerization;
- **"Porous":** refers to a mineral compound comprising mesopores (pore size from 2 nm to 50 nm, preferably from 2 and 30 nm), micropores (pore size lower or equal to 2 nm) and/or macropores (pore size equal or higher than 50 nm). In the present invention, porous compound preferably refers to mineral compound comprising mesopores with a pore size ranging from 2 and 30 nm. In the present invention, porous compound preferably refers to a non-sintered porous nanoparticle; more preferably to a porous silica nanoparticle. The mesopores may have different structures: cubic, spherical and/or wormlike;
- **"Resin matrix":** refers to any natural or synthetic organic blend consisting of noncristalline or viscous substance able of hardening permanently;
- "**Vinylic**"**:** refers to a CH₂=CH- group;
- **"w/w":** in weight to the total weight of the composition, unless otherwise specified.

### DETAILED DESCRIPTION

This invention relates to a composition resulting from the mixture of a resin matrix comprising photopolymerizable monomers and a fillers blend comprising:
▪ at least one primary filler selected from mineral compounds; and
▪ at least one porous filler; preferably selected from porous silica particles, more preferably selected from SBA-15, MCM-48, MSU-H and MCM-41;
wherein the ratio between the resin matrix and the fillers blend ranges from 0.25 to 2.5 ; preferably is 0.33; the porous filler has a size ranging from 20nm to 20µm and a pore size ranging from 2nm to 30nm.

Especially, the invention relates to a composition resulting from the mixture of:
- a resin matrix comprising:
   ▪ from 57% to 99% w/w of a mixture of photopolymerizable monomers in weight of the total weight of the resin matrix;
   ▪ from more than 0% to 42% w/w of at least one plasticizer, in weight of the total weight of the resin matrix;
   ▪ from more than 0% to 1.12% w/w of at least one polymerization inhibitor;
   ▪ from more than 0% to 3% w/w of at least one photoiniator in weight of the total weight of the resin matrix;
- and a fillers blend comprising:
   ▪ at least one primary filler selected from mineral compounds; and
   ▪ from more than 0 to 30% w/w at least one porous filler in weight of the total weight of the fillers blend; preferably selected from porous silica particles, more preferably selected from SBA-15, MCM-48, MSU-H and MCM-41;
wherein the ratio between the resin matrix and the fillers blend ranges from 0.25 to 2.5 ; preferably is 0.33;
the porous filler has a size ranging from 20nm to 20µm and a pore size ranging from 2nm to 30nm.

According to one embodiment, the photopolymerizable monomers are selected from the group comprising at least one acrylate, alkylacrylate, methacrylate, styrenic, vinylic, allylic, oxetan, alkyloxetan, cyclic ether, acrylamide, methacrylamide and derivatives thereof. According to one preferred embodiment, the photopolymerizable monomers comprise at least one methacrylate group and derivatives thereof. According to one preferred embodiment, the photopolymerizable monomers are monomers having no methacrylate function. According to one preferred embodiment, the resin matrix further comprises monomers having a carbamate function. According to one preferred embodiment, the photopolymerizable monomers comprise carbamate-alkylacrylate monomer.

This invention also relates to a composition resulting from the mixture of:
- a resin matrix comprising:
   ▪ from 57% to 99%w/w of a mixture of photopolymerizable monomers comprising at least one carbamate-alkylacrylate monomer in weight of the total weight of the resin matrix;
   ▪ from more than 0% to 42%w/w of at least one plasticizer, in weight of the total weight of the resin matrix;
   ▪ from more than 0% to 1.12%w/w of at least one polymerization inhibitor in weight of the total weight of the resin matrix;
   ▪ from more than 0% to 3%w/w of at least one photoiniator in weight of the total weight of the resin matrix;
- and a filler blend comprising:
   ▪ at least one primary filler selected from mineral compounds; and
   ▪ from more than 0 to 30%w/w at least one porous filler in weight of the total weight of the filler blend; preferably selected from porous silica particles, more preferably selected from SBA-15, MCM-48, MSU-H and MCM-41;
wherein the ratio between the resin matrix and the filler blend ranges from 0.25 to 2.5, preferably is 0.33;
the porous filler has a size ranging from 20nm to 20µm and a pore size ranging from 2nm to 30nm.

According to a first embodiment, the composition does not comprise any solvent.

According to a first embodiment, the composition further comprises a solvent selected from water or organic solvent such as but not limited to hexane, ether petroleum, benzene, toluene or xylene; chlorinated hydrocarbons, such as trichloroethylene, 1,2-dichloroethane, tetrachloromethane, trifluoromethylbenzene, chloroform or dichloromethane; alcohols, such as methanol, ethanol, isopropanol, n-propanol, n-butanol or tert-butanol; ethers such as diethyl ether, diisopropyl ether, tetrahydrofurane (THF) or dioxane; glycol ethers such as ethylene glycol monomethyl ou monoethyl ether or ethylene glycol dimethyl ether (diglyme); ketones, such as acetone or butanone; amides, such as acetamide, dimethylacetamide, N-methylpyrrolidone (NMP) or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate and mixture thereof.

According to one embodiment, the composition further includes at least one pigment; preferably metal oxide. According to one embodiment, said pigment may be iron metal oxide. Advantageously, said iron oxide may be selected from iron oxide yellow, red and brown. In one embodiment, the composition may further include a mixture of dyes. In one embodiment, the composition may further include a mixture of iron oxides.

According to one embodiment, the composition has stickiness properties. These stickiness properties are measured by a traction-compression test (W, in mJ). In one embodiment, the stickiness value is ranging from 0,5 mJ to 5 mJ; preferably is below 2mJ; more preferably is below 1.5mJ.

According to one embodiment, the resin matrix comprises photopolymerizable monomers comprising at least one carbamate-alkylacrylate monomer. By "carbamate-alkylacrylate monomer", is meant any compound having both a carbamate and an alkylacrylate function; preferably the carbamate-alkylacrylate monomer is a carbamate-methacrylate monomer. According to a one embodiment, the resin matrix comprises from 57% to 99%w/w, preferably from 60% to 81%w/w in weight of the total weight of the resin matrix of a mixture of photopolymerizable monomers comprising at least one carbamate-alkylacrylate monomer.

According to one embodiment, the resin matrix further comprises at least one monomer selected from the group comprising at least one acrylate, alkylacrylate, methacrylate, styrenic, vinylic, allylic, oxetan, alkyloxetan, cyclic ether, acrylamide, methacrylamide and derivatives thereof. According to one preferred embodiment, the resin matrix further comprises methacrylates and derivatives thereof. According to one preferred embodiment, the resin matrix further comprises monomers having no methacrylate function. According to one preferred embodiment, the resin matrix further comprises monomers having a carbamate function.

According to the invention, preferred carbamate-methacrylate monomer is selected from 1,6-hexanedicarbamatedimethacrylate (HDCDMA), ((((methylenebis(cyclohexane-4,1-diyl))bis(azanediyl))bis(carbonyl))bis(oxy))bis(ethane-2,1-diyl) bis(2-methylacrylate) (DCyDCDMA), 2-((((5-(((2-(methacryloyloxy)ethoxy)carbonyl)amino)-1,3,3-trimethylcyclohexyl)methyl)carbamoyl)oxy)ethyl methacrylate (IPDCDMA), (Z)-2-(((6-(5-(6-(((2-((3-methylbuta-1,3-dien-2-yl)oxy)ethoxy)carbonyl)amino)hexyl)-6-((6-(((2-((3-methylbuta-1,3-dien-2-yl)oxy)ethoxy)carbonyl)amino)hexyl)imino)-2,4-dioxo-1,3,5-oxadiazinan-3-yl)hexyl)carbamoyl)oxy)ethyl methacrylate (DXPTCTMA), ((((((2-oxo-1,3,5-triazinane-1,3,5-triyl)tris(methylene))tris(3,3,5-trimethylcyclohexane-5, 1-diyl))tris(azanediyl))tris(carbonyl))tris(oxy))tris(ethane-2,1-diyl) tris(2-methylacrylate) (DZTCTMA), (((((5-(6-(3-(3-(((2-(methacryloyloxy)ethoxy)carbonyl)amino)-4-methylphenyl)-5-(6-(((2-(methacryloyloxy)ethoxy)carbonyl)amino)hexyl)-2,4,6-trioxo-1,3,5-triazinan-1-yl)hexyl)-2,4,6-trioxo-1,3,5-triazinane-1,3-diyl)bis(2-methyl-5,1-phenylene))bis(azanediyl))bis(carbonyl))bis(oxy))bis(ethane-2,1-diyl) bis(2-methylacrylate) (DHLTetCTetMA), 2,15-bis((4-(tert-butyl)phenoxy)methyl)-4,13-dioxo-3,14-dioxa-5,12-diazahexadecane-1,16-diyl bis(2-methylacrylate) (DTPHDMA), ((((methylenebis(cyclohexane-4,1-diyl))bis(azanediyl))bis(carbonyl))bis(oxy))bis(3-(4-(tert-butyl)phenoxy)propane-2,1-diyl) bis(2-methylacrylate) (DTPDCHDMA), 4,13-dioxo-2,15-bis(phenoxymethyl)-3,14-dioxa-5,12-diazahexadecane-1,16-diyl bis(2-methylacrylate) (DPHDMA), 2-((4-(tert-butyl)phenoxy)methyl)-4,13-dioxo-15-(phenoxymethyl)-3,14-dioxa-5,12-diazahexadecane-1,16-diyl bis(2-methylacrylate) (TBPPHDMA), 3-(4-(tert-butyl)phenoxy)-2-(((4-((4-((((1-(methacryloyloxy)-3-phenoxypropan-2-yl)oxy)carbonyl)amino)cyclohexyl)methyl)cyclohexyl)carbamoyl)oxy)propyl methacrylate (TBPPDCHDMA), 2,15-bis((3,5-di-tert-butylphenoxy)methyl)-4,13-dioxo-3,14-dioxa-5,12-diazahexadecane-1,16-diyl bis(2-methylacrylate) (TTPHDMA), ((((methylenebis(cyclohexane-4,1-diyl))bis(azanediyl))bis(carbonyl))bis(oxy))bis(3-(3,5-di-tert-butylphenoxy)propane-2,1-diyl) bis(2-methylacrylate) (TTPDCHDMA), 2-(((4-((4-((((1-(methacryloyloxy)-3-phenoxypropan-2-yl)oxy)carbonyl)amino)cyclohexyl)methyl)cyclohexyl)carbamoyl)oxy)ethyl methacrylate (HDCHDMA), 3-(((4-((4-((((1-(methacryloyloxy)-3-phenoxypropan-2-yl)oxy)carbonyl)amino)cyclohexyl)methyl)cyclohexyl)carbamoyl)oxy)propyl methacrylate (PDCHDMA), 2,15-bis((4-(tert-butyl)phenoxy)methyl)-8,10,10-trimethyl-4,13,18-trioxo-3,14,17-trioxa-5,12-diazaicos-19-en-1-yl methacrylate (TMTBPNHDMA), 15-((4-(tert-butyl)phenoxy)methyl)-8,10,10-trimethyl-4,13,18-trioxo-2-(phenoxymethyl)-3,14,17-trioxa-5,12-diazaicos-19-en-1-yl methacrylate (TMPNHDMA).

According to one preferred embodiment, the carbamate-methacrylate monomer is 15-((4-(tert-butyl)phenoxy)methyl)-8,10,10-trimethyl-4,13,18-trioxo-2-(phenoxymethyl)-3,14,17-trioxa-5,12-diazaicos-19-en-1-yl methacrylate (TMPNHDMA).

According to a second embodiment, the mixture of photopolymerizable monomers consists of carbamate-alkylacrylate monomers; preferably carbamate-methacrylate monomers. According to one embodiment, the mixture of photopolymerizable monomers comprises carbamate-alkylacrylate monomers; preferably similar or different carbamate-methacrylate monomers.

According to one embodiment, the mixture of photopolymerizable monomers comprises at least one carbamate-alkylacrylate monomer and at least one monomer which does not comprises alkylacrylate function; preferably the mixture of photopolymerizable monomers comprises at least one carbamate-methacrylate monomer and at least one monomer which does not comprises methacrylate function.

According to one embodiment, the resin matrix comprises a plasticizer selected from compounds having at least two methacrylate functions and no carbamate function, preferably 1,6 hexanediol dimethacrylate (HDDMA) or trimethylpropyltrimethacrylate (TMPTMA). According to one embodiment, the resin matrix comprises from more than 0% to 42%w/w of at least one plasticizer; preferably from 15% to 36%w/w in weight of the total weight of the resin matrix.

According to one embodiment, the resin matrix comprises at least one polymerization inhibitor, preferably the polymerization inhibitor is selected from butylhydroxytoluene (BHT). According to one embodiment, the resin matrix comprises at least one polymerization inhibitor selected from antioxidants. According to one embodiment, the resin matrix comprises from more than 0% to 2%w/w of at least one polymerization inhibitor; preferably from 0.01% to 0.9% w/w; more preferably from 0.05% to 0.3% w/w in weight of the total weight of the resin matrix.

According to one embodiment, the resin matrix comprises at least one photoiniator selected from 2-tert-butylanthraquinone; camphorquinone; diphenyl(2,4,6-trimethylbenzoyl)phosphine oxide; 9,10-phenanthrenequinone; phenylbis(2,4,6-trimethylbenzoyl)phosphine oxide; 2-benzyl-2-(dimethylamino)-4'-morpholinobutyrophenone; 3,6-bis(2-methyl-2-morpholinopropionyl)-9-octylcarbazole; 4'-tert-butyl-2',6'-dimethylacetophenone; 2,2-diethoxyacetophenone; 4'-ethoxyacetophenone; 3'-hydroxyacetophenone; 4'-hydroxyacetophenone; 1-hydroxycyclohexylphenylcetone; 2-hydroxy-4'-(2-hydroxyethoxy)-2-methylpropiophenone; 2-hydroxy-2-methylpropiophenone; 2-methyl-4'-(methylthio)-2-morpholinopropiophenone; 4'-phenoxyacetophenone; benzoin; benzoinethyl ether benzoinmethyl ether; 4,4'-dimethylbenzoin, 4,4'-dimethylbenzil; benzophenone; benzoylbiphenyl; 4,4'-bis(diethylamino)benzophenone; 4,4'-dihydroxybenzophenone; 3,4-dimethylbenzophenone; 3-hydroxybenzophenone; 4-hydroxybenzophenone; 2-methylbenzophenone; 3-methylbenzophenone; 4-methylbenzophenone; methylbenzoylformate; Michler ketone; 1-chloro-4-propoxy-9H-thioxanthen-9-one; 2-chlorothioxanthen-9-one; 2,4-diethyl-9H-thioxanthen-9-one; isopropyl-9H-thioxanthen-9-one; 10-methylphenothiazin; thioxanthen-9-one; preferably from 2,4-dihydroxybenzophenone, ethyl 4-dimethylaminobenzoate and camphorquinone. According to one embodiment, the resin matrix comprises all three 2,4-dihydroxybenzophenone, ethyl 4-dimethylaminobenzoate and camphorquinone. According to one embodiment, the resin matrix comprises from more than 0% to 3%w/w of at least one photoinitiator; preferably from 1% to 2.5% w/w in weight of the total weight of the resin matrix.

According to another embodiment, the filler blend comprises at least one primary filler selected from mineral compounds. According to the invention, by "mineral compounds" is meant all compounds which are not organic. According to one embodiment, the filler blend comprises at least one primary filler.

Preferred primary fillers are non-porous silica beads, non-porous ceramic beads, glass beads and/or zirconium oxide non-porous particles; preferably non-porous silica beads, barium glass and Schott Glass^{®} beads; more preferably Schott Glass^{®} SG-8235; SG-YBF-10-4-3%Sil or SG-YBS030CMP10 glass.

The beads may or may not be spherical. In a preferred embodiment, the mean diameter of the primary fillers is less than 10 µm, preferably is ranging from 0.3 to 5 µm, more preferably is about 0.7 µm.

According to one embodiment, the filler blend comprises at least one porous filler selected from porous mineral compounds; preferably selected from porous mineral particles; more preferably selected from silica particles; more preferably selected from SBA, SBA-15, MCM-48, MSU-H and MCM-41. According to one embodiment, the filler blend comprises from more than 0 to 20% w/w, preferably from 1 to 3% w/w in weight to the total weight of the filler blend.

The porous fillers may or may not be spherical. In a preferred embodiment, the porous fillers are spherical. In a preferred embodiment, the mean diameter of the porous fillers has a size ranging from 20nm to 20µm, preferably ranging from 30 to 300 nm, more preferably from 40 to 100 nm.

In an embodiment, the porous fillers included in the composition of the invention have a pore size ranging from 2nm to 50nm, preferably from 4nm to 30nm. In an embodiment, the porous fillers included in the composition of the invention have a pore size ranging from 2nm to 30nm. According to one embodiment, the structure of pore size may be hexagonal, wormlike or cubic.

In an embodiment, the porous fillers are functionalized; preferably by a silane bearing at least one functional or hydrophobic group selected from methacryloyl, methacryloylalkyl, aminoalkyl, alkoxy, vinyl, allyl, aryl or alkyl. According to one preferred embodiment, the porous fillers are functionalized by a silane bearing at least one methacryloyl or methacryloylalkyl group. According to one preferred embodiment, the porous fillers are functionalized by 3-methacryloxypropyltrimethyoxysilane (MPTS). According to one preferred embodiment, the porous fillers are functionalized by a silane bearing at least one tetrafluoroethylene group.

According to one preferred embodiment, the porous fillers are prepolymerized filler particles; preferably semi organic prepolymerized fillers.

The invention also relates to a method of preparing the composition of the invention comprising a step of mixing of a resin matrix and a filler blend, wherein the resin matrix comprises:
- from more than 0% to 2% w/w of at least one polymerization inhibitor ;
- from more than 0% to 3% w/w of photoinitiator in weight of the total resin matrix;
- from more than 0% to 42% w/w of at least one plasticizer, and
- from 57% to 99% w/w of a mixture of photopolymerizable monomers comprising carbamate-alkylacrylate monomers in weight of the total resin matrix;
and the filler blend comprises at least one primary filler and at least one porous filler;
wherein
said porous filler is preferably selected from porous silica particles, more preferably selected from SBA-15, MCM-48, MSU-H and MCM-41;
said porous filler has a size ranging from 20nm to 20µm and a pore size ranging from 2nm to 30nm;
the content of porous filler is ranging from more than 0% to 30%, preferably from 1 to 3% in weight of the total weight of the filler in the composition.

According to one embodiment, the composition further includes at least one pigment; preferably metal oxide. According to one embodiment, said pigment may be iron metal oxide. Advantageously, said iron oxide may be selected from iron oxide yellow, red and brown. In one embodiment, the composition may further include a mixture of dyes. In one embodiment, the composition may further include a mixture of iron oxides.

The invention also relates to a process for manufacturing a composite comprising:
(a) preparing the composition of the invention as described above;
(b) molding the composition resulting from the step (a);
(c) implementing photopolymerization.

According to one embodiment, the composition is hand-molded or molded with the help of an apparatus.

According to one embodiment, the photopolymerization is initiated by irradiation. In one embodiment, the initiation of photopolymerization is carried out with a primary light source. In one embodiment, the initiation of photopolymerization is carried out with a secondary light source. In one embodiment, the initiation of photopolymerization is carried out with a filtered light source. In one embodiment, the initiation of photopolymerization is carried out with the sunlight. In one embodiment, the initiation of photopolymerization is carried out at a wavelength ranging from 330 to 500nm; preferably the initiation of photopolymerization is carried out at 470nm.

According to one embodiment, the photopolymerization is implemented during a time ranging from 5 to 60s; preferably ranging from 10s to 40s; more preferably during 20s.

According to one embodiment, the photopolymerization provides a cured composition.

The invention also relates to a kit of parts for the preparation of a composition suitable for use in dentistry, comprising in a first container a resin matrix as described above and a second container a filler blend comprising from at least one primary filler and at least one porous filler.

According to one embodiment the first container allows keeping resin matrix protected from light.

The invention also relates to compositions of the invention for use in the field of dentistry; especially in dental sealing and/or restoration.

According to one embodiment, the composition of the invention is useful for providing dental compositions.

According to one embodiment, the composition of the invention is useful for providing a composite; preferably a dental composite.

### BRIEF DESCRIPTION OF THE DRAWINGS

**Figure 1** is a graph showing the evolution of spread diameter (mm) of molded compositions in function of the porous filler content (%w/w of SBA-MPTS).
**Figure 2A** is a histogram showing the effect of the progressive incorporation of porous fillers in a composite material on stickiness and consistency.
**Figure 2B** is a graph showing the evolution of the stickiness (in mJ) in function of the porous filler content (%w/w of SBA-MPTS).
**Figure 3** is a graph showing the evolution of the stickiness (in mJ) in function of the nature and the functionalization of the porous filler.

### EXAMPLES

The present invention is further illustrated by the following examples.

### Abbreviations

BET: Brunauer-Emmett-Teller (BET) method
EtOH: ethanol
HDMS: hexamethyldisilane
MPTS: 3-methacryloxypropyltrimethyoxysilane
kg: kilogram
mL: milliliter
min: minute
nm: nanometer
rpm: round per minute
SEM: Scanning Electron Microscopy

### Materials

All the reagents were commercially purchased (Aldrich, ABCR or Carlo Erba) and used without any further purification.

### Methods

### Porous fillers synthesis (SBA)

A solution of Pluronic P-123 (4g) in deionized water (126 mL) was mixed with a solution of HCl (37%wt.). A micellar solution is obtained. After heated the micellar solution previously prepared, is added 9,1 mL of tetraethylorthosilicate. The reactive mixture is stirred during 24 hours at 35°C and then the suspension is separated in several plastic plots for heating at 90°C during 24 hours. Then, particles were removed of the solution by filtration and washed three times with water. The particles were finally dried at 80°C for 6 hours and crushed in a mortar.

In order to remove surfactant of the particles pores, said particles are first heated from 25°C to 550°C in 7 hours, then hold for 5 hours at 550°C and finally cooled to 25°C in 7 hours.

The obtained porous particles are crushed and stored under inert atmosphere. The particles size is 800 nm. The pore size of the obtained porous particles is 7 nm with a hexagonal structure.

The obtained porous particles were characterized by BET, SEM and pycnometer. The obtained porous particles have a specific area about 824 m²/g, a size of 800 nm and a pore volume about 0.9 cm³/g.

### Functionalization of porous fillers

1 g of porous filler was dispersed in toluene (100mL). The dispersion is heated at 80°C; then 5 mL of MPTS were added. The reactional mixture is stirred at 80°C during 12h. After the reaction, the obtained functionalized particles were washed three times by centrifugation-redispersion cycles in EtOH (centrifugation step: during 5 min at 7 000 rpm). Finally, the functionalized porous particles are dried 12h at 80°C; then ground in a mortar and kept under inert atmosphere for avoiding moisture adsorption.

### Consistency test

A fixed quantity (0.15 cm³) of the composition of the invention is molded with a 1 ml-syringe having an output piston of diameter 4.5 mm. Then, the sample is placed between two foils and a load (1,5 kg) is applied on the sample for 3 min. At the end of the consistency test, the resulting spread diameter of the sample is measured.

Higher the consistency, lower the spread diameter.

This test is realized at room temperature.

### Example 1: Examples of compositions

Compositions of the invention were obtained by mixing a resin matrix (Table 1) and a filler blend (Table 2).

**Table 1. Examples of resin matrix.**

| Components of resin matrix | Quantity for 100 g of resin matrix | |
|---|---|---|
| | Composition # | |
| | **A** | **B** |
| Carbamate-alkylacrylate monomers | 56.8 | 57 |
| Other monomers | 24.13 | 5 |
| Plasticizer | 17.60 | 35.66 |
| Polymerization inhibitor | 0.09 | 0.23 |
| Photoinitiator | 1.38 | 2.11 |

**Table 2. Examples of filler blend.**

| Components of filler blend | | Quantity for 100 g of filler blend | | | | | | | |
|---|---|---|---|---|---|---|---|---|---|
| | | Filler blend # | | | | | | | |
| | | **1** | **2** | **3** | **4** | **5** | **6** | **7** | **8** |
| **Primary filler** | Barium glass | 100 | 90 | 80 | 70 | 0 | 0 | 0 | 0 |
| | SG-8235 | | | | | | | | |
| | (0.7µm; 9.4% MPTS) | | | | | | | | |
| | Barium glass | 0 | 0 | 0 | 0 | 85 | 84.15 | 83.3 | 82.45 |
| | SG-8235 | | | | | | | | |
| | (0.4µm; 9.4% MPTS) | | | | | | | | |
| | Ytterbium glass Yb₂O₃-SiO₂ | 0 | 0 | 0 | 0 | 10 | 9.9 | 9.8 | 9.7 |
| | SG-YBF-10-4-3%MPTS | | | | | | | | |
| | Ytterbium glass Yb₂O₃-SiO₂ | 0 | 0 | 0 | 0 | 1.5 | 1.49 | 1.47 | 1.46 |
| | SG-YBS030CMP10 | | | | | | | | |
| | (6.5µm; 10% MPTS) | | | | | | | | |
| **Porous filler** | Aerosil OX 50 PA-Sil | 0 | 0 | 0 | 0 | 3.5 | 3.47 | 3.43 | 3.40 |
| | SBA functionalized with 10%wt. MPTS | 0 | 10 | 20 | 30 | 0 | 1 | 2 | 3 |

**Table 3. Examples of compositions from resin matrix A.**

| Components of compositions | Composition # | | | | | | | | |
|---|---|---|---|---|---|---|---|---|---|
| | **A**-**1** | **A**-**2** | **A**-**3** | **A**-**4** | **A**-**5** | **A**-**6** | **A**-**7** | **A**-**8** | **A**-**9** |
| **Resin matrix** | A | A | A | A | A | A | A | A | A |
| **Filler blend** | 1 | 2 | 3 | 4 | 5 | 6 | 7 | 8 | 9 |
| **Ratio resin matrix**/ **fillers blend** | 0.35 | 0.52 | 0.67 | 0.85 | 0.28 | 0.27 | 0.27 | 0.27 | 2.33 |
| **Fillers (% in weight to the total weight of the composition)** | 74 | 66 | 60 | 54 | 78.4 | 78.6 | 78.8 | 79 | 30 |

**Table 4. Examples of compositions from resin matrix B.**

| Components of compositions | Composition # | | | |
|---|---|---|---|---|
| | **B**-**1** | **B**-**2** | **B**-**3** | **B**-**4** |
| **Resin matrix** | B | B | B | B |
| **Filler blend** | 1 | 2 | 3 | 4 |
| **Ratio resin matrix/ filler blend** | 0.35 | 0.51 | 0.65 | 0.54 |
| **Fillers (% in weight to the total weight of the composition**) | 74.2 | 66.4 | 60.6 | 65 |

### Example 2: Effect of the incorporation of porous fillers in a resin matrix

It is known by the skilled human in the art, that increasing the fillers content in a composition leads to increase its viscosity and its consistency.

These experiments aim to prove that for the same fillers content, compositions comprising porous fillers are more fluid *(i.e.* low consistency or high spread diameter) than compositions with non-porous fillers.

Different compositions were synthetized by mixing primary fillers (Schott Glass SG-8235) with porous fillers functionalized with 3-methacryloxypropyltrimethyoxysilane (SBA-MPTS) (see Table 2, filler blend 1-4) into the same resin matrix A as described above.

This study focuses on compositions comprising a mixture SG-8235/SBA-MPTS with the following ratios: 100/0; 90/10; 80/20 and 70/30, *i.e.* comprising 0%, 10%, 20% or 30% in weight of porous filler in the total weight of filler blend respectively.

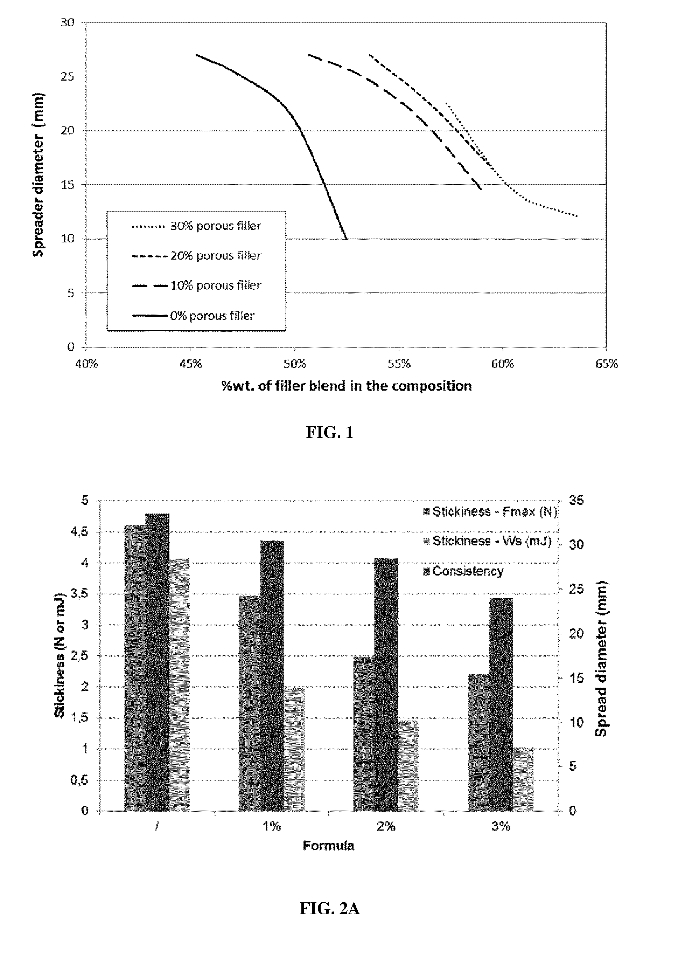

The evolution of the spread diameter for these different compositions in function of the filler content in the composition is shown **Figure 1****.**

Practically, if the spread diameter ranges:
- between 8mm and 15mm : composition is considered too hard;
- between 15mm and 20mm : the consistency of composition is considered as being improved compared to current commercial compositions;
- between 20mm and 26mm : the consistency of composition is considered as being comparable to current commercial compositions;
- higher than 26mm: composition is considered too fluid.

Unexpectedly, the results show that:
- for compositions without any porous fillers *(i.e.* 0% of porous filler), the spread diameter dramatically decreases when the fillers content is higher than 50%;
- when compared at the same fillers content in the composition, the spread diameter of compositions comprising 10-30% w/w of porous fillers is much higher than compositions without any porous fillers;
- when compared at the same value of spread diameter, compositions comprising porous fillers (10-30%) correspond to compositions with much higher fillers content than compositions without any porous fillers.

In conclusion, these experiments show that the incorporation of porous fillers in a composition allows obtaining (compared to compositions comprising non-porous fillers): 1) at equal fillers content, more fluid materials; 2) at equal spread diameter *(i.e.* at the same consistency), composition with higher fillers content.

### Example 3: Effect of incorporation of low content of porous filler (1%-3%wt.) in a composite

The consistency and the stickiness of a composite are important parameters that the practitioner has to take into account for using said composite; for example, in the dental field. Indeed, a composition of said composite too sticky and/or too consistent could not be easily handled.

These experiments aim to show the effect of a low content of porous filler in a composite on its consistency and its stickiness.

Three compositions were obtained by mixing the same resin matrix A (see Table 1) with a filler blend comprising 1%, 2% or 3% w/w of SBA-MPTS (see Table 2, filler blend 6, 7 and 8), in weight of the total filler blend.

The spread diameter (mm) and the stickiness were measured for each obtained composite.

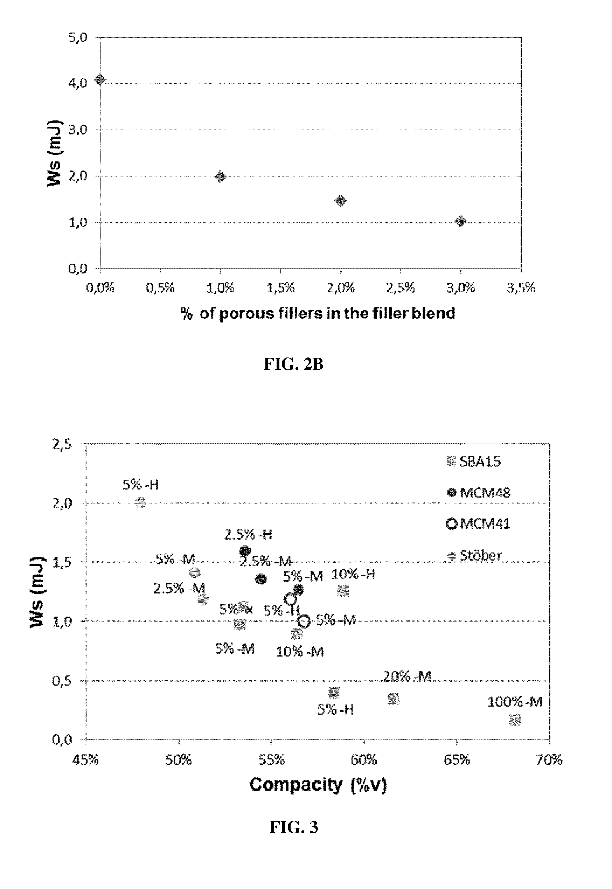

**Figures 2A** and **2B** show the results for compositions comprising 1-3% w/w of porous fillers compared to compositions comprising non porous fillers (reference).

First, the results show that the incorporation from 1% to 3% w/w of porous fillers in composite, allows strongly decreasing the stickiness.

Furthermore, it can be noticed that the consistency of composites comprising porous filler also decreases compared to composite comprising non-porous fillers.

In conclusion, the incorporation of a low content of porous fillers enables to enhance the handling properties for the practitioner.

### Example 4: Effect of incorporation of porous filler (2.5%-20%wt.) in a composite on the stickiness of final material

These experiments aim to compare the effect of different porous fillers functionalized or not, in a composite on its consistency and its stickiness.

Different compositions (Table 5) were synthetized by mixing several kinds of porous fillers into the same primary filler (Barium Glass, SG-8235) with the same resin matrix A as described above.

The porous fillers are selected from SBA-15, MCM-48, MCM-41 or Stöber particles functionalized or not with MPTS (*i.e*. hydrophilic moiety) or HMDS (*i.e.* hydrophobic moiety).

**Table 5. Compositions for stickiness tests.**

| **Composition N°** | **Porous filler** | | | **Filler rate** | **Compacity** |
|---|---|---|---|---|---|
| | ***Type*** | ***Grafting*** | ***%w¹*** | ***%w²*** | ***%v*** |
| **i** | SBA15 | MPTS | 10 | 65.0 | 55.5 |
| **ii** | SBA15 | none | 5 | 69.0 | 53.5 |
| **iii** | SBA15 | HMDS | 5 | 72.8 | 58.4 |
| **iv** | SBA15 | HMDS | 10 | 68.5 | 58.9 |
| **v** | SBA15 | MPTS | 5 | 69.3 | 53.3 |
| **vi** | SBA15 | MPTS | 10 | 69.9 | 56.4 |
| **vii** | SBA15 | MPTS | 20 | 60.8 | 61.6 |
| **viii** | SBA15 | MPTS | 100 | 33.6 | 68.1 |
| **ix** | MCM48 | HMDS | 2.5 | 72.5 | 53.6 |
| **x** | MCM48 | MPTS | 2.5 | 73.1 | 54.4 |
| **xi** | MCM48 | MPTS | 5 | 72.4 | 56.4 |
| **xii** | MCM41 | HMDS | 5 | 70.5 | 56.0 |
| **xiii** | MCM41 | MPTS | 5 | 71.0 | 56.7 |
| **xiv** | Stöber | HMDS | 5 | 70.0 | 48.0 |
| **xv** | Stöber | MPTS | 2.5 | 72.9 | 51.3 |
| **xvi** | Stöber | MPTS | 5 | 72.3 | 50.9 |

| | | | | | |
|---|---|---|---|---|---|
| *¹to the total weight of the porous particles, ²to the total weight of the composition.* | | | | | |

The stickiness properties of these compositions were studied.

The results presented **Figure 3****,** show that 1) at the same compacity, compositions comprising SBA-15 particles are the lowest stickiness compositions; and that 2) the stickiness properties of the compositions of the present invention may be modulated both by the kind of functionalization and by the load of the porous fillers in the filler blend.

## Claims

1. A composition resulting from the mixture of :
- a resin matrix comprising:
▪ from 57% to 99%w/w of a mixture of photopolymerizable monomers comprising at least one carbamate-alkylacrylate monomer;
▪ from more than 0% to 42%w/w of at least one plasticizer;
▪ from more than 0% to 2%w/w of at least one polymerization inhibitor ;
▪ from more than 0% to 3% w/w of at least one photoiniator;
in weight of the total weight of the resin matrix;
- and a filler blend comprising:
▪ at least one primary filler selected from mineral compounds; and
▪ from more than 0 to 30%w/w at least one porous filler, preferably selected from porous silica particles, more preferably selected from SBA-15, MCM-48, MSU-H and MCM-41;
in weight of the total weight of the filler blend;
wherein
the ratio between the resin matrix and the filler blend ranges from 0.25 to 2.5; preferably is 0.33;
the porous filler has a size ranging from 20nm to 20µm and a pore size ranging from 2nm to 30nm.

2. A composition according to claim **1,** wherein said composition does not comprise any solvent.

3. A composition according to anyone of claims **1** or **2,** wherein the resin matrix further comprises at least one monomer selected from the group comprising at least one acrylate, alkylacrylate, methacrylate, styrenic, vinylic, allylic, oxetan, alkyloxetan, cyclic ether, acrylamide, methacrylamide and derivatives thereof.

4. A composition according to anyone of claims **1** to **3,** wherein the plasticizer is selected from compounds having at least two methacrylate functions and no carbamate function; preferably the plasticizer is 1,6 hexanediol dimethacrylate (HDDMA) or trimethylpropyltrimethacrylate (TMPTMA).

5. A composition according to anyone of claims **1** to **4,** wherein the polymerization inhibitor is butylhydroxytoluene.

6. A composition according to anyone of claims **1** to **5,** wherein the photoinitiator is a mixture of 2,4-dihydroxybenzophenone, ethyl 4-dimethylaminobenzoate and camphorquinone.

7. A composition according to anyone of claims **1** to **6,** wherein the primary filler is a Schott Glass^{®}; preferably a barium glass, an ytterbium glass or a mixture thereof.

8. A composition according to anyone of claims **1** to **7,** wherein the porous filler is functionalized by at least one silane bearing one or more functional or hydrophobic groups; preferably said groups are selected from methacryloyl, methacryloylalkyl, aminoalkyl; alkoxy, vinyl, allyl, aryl or alkyl.

9. A method of preparing the composition according to anyone of claims **1** to **8,** comprising a step of mixing of a resin matrix and a filler blend, wherein the resin matrix comprises:
- from more than 0% to 2% w/w of at least one polymerization inhibitor ;
- from more than 0% to 3% w/w of photoinitiator in weight of the total resin matrix;
- from more than 0% to 42% w/w of at least one plasticizer, and
- from 57% to 99% w/w of a mixture of photopolymerizable monomers comprising carbamate-alkylacrylate monomers in weight of the total resin matrix;
and the filler blend comprises at least one primary filler and at least one porous filler;
wherein
said porous filler is preferably selected from porous silica particles, more preferably selected from SBA, MCM-48, MSU-H and MCM-41;
said porous filler has a size ranging from 20nm to 20µm and a pore size ranging from 2nm to 30nm;
the content of porous filler is ranging from more than 0% to 30%, preferably from 1 to 3% in weight of the total weight of the filler blend in the composition.

10. A process for manufacturing a composite comprising:
(a) preparing the composition according to anyone of claims **1** to **8;**
(b) molding the composition resulting from the step (a);
(c) implementing photopolymerization.

11. The process according to claim **10,** wherein photopolymerization is carried out during a time ranging from 5 to 60s, preferably ranging from 10s to 40s; more preferably during 20s.

12. A kit of parts for the preparation of a composition according to anyone of claims **1** to 8 suitable for use in dentistry, comprising in a first container a resin matrix and a second container a filler blend comprising at least one primary filler and at least one porous filler.

13. The composition according to anyone of claims **1** to **8** for use in dental sealing and/or restoration.

14. The composition for use according to claim **13** in the manufacture of a dental composite.
